# EUROPEAN PATENT APPLICATION

(11) **EP 3 943 097 A1**
(43) Date of publication of application: **26.01.2022**
(21) Application number: 20187684.4
(22) Date of filing: 24.07.2020
(51) Int. Cl.: A61K 38/00, A61K 39/00, A61K 39/395, A61P 35/00, A61P 37/06, C07K 16/24

(54) **CAR-T CELL THERAPY**

(71) Applicant: AB2 Bio SA, 1015 Lausanne (CH)
(72) Inventor: The designation of the inventor has not yet been filed
(74) Representative: Vossius & Partner Patentanwälte Rechtsanwälte mbB

(57) **Abstract**

The present invention provides compounds and compositions for use in the treatment of cancer as part of a Chimeric Antigen Receptor T-cell (CAR-T) therapy. In particular, the present invention discloses IL-18 binding molecules such as, for example, the IL-18 binding protein (IL-18BP) for use in Chimeric Antigen Receptor T-cell (CAR-T) therapy as a modulator to avoid or minimize side effects.

## Description

The present invention provides compounds and compositions for use in the treatment of cancer as part of a Chimeric Antigen Receptor T-cell (CAR-T) therapy. In particular, the present invention provides IL-18 binding molecules such as, for example, the human IL-18 binding protein (IL-18BP), for use in Chimeric Antigen Receptor T-cell (CAR-T) therapy as a modulator to avoid or minimize undesired side effects.

Adoptive T cell transfer (ACT) is a new area of transfusion medicine involving the infusion of lymphocytes to mediate antitumor, antiviral, or anti-inflammatory effects. The field has rapidly advanced from a promising form of immuno-oncology in preclinical models to the recent commercial approvals of chimeric antigen receptor (CAR) T cells to treat leukemia and lymphoma.

A CAR combines antigen-binding domains, most commonly, a single-chain variable fragment (scFv) derived from the variable domains of antibodies with the signaling domains of the "TCR" chain and additional costimulatory domains from receptors such as CD28, OX40, and CD137.

The results from the initial clinical trials using first-generation CAR designs in patients with various cancers were disappointing. However, in 2011, second-generation CAR-T cells targeting CD19 and encoding costimulatory domains emerged as the lead paradigm for engineered T cell therapies in cancer. Early results from CAR-T cell trials evaluating other targets indicated that the CD19 offtumor cross-reactions are not a singular example, but may be generally observed with other lineage dependent targets.

CAR-T technology has now been shown to have broader applications beyond CD19, and early phase clinical trials of CAR-T cells targeting BCMA and CD22 have reported similarly potent antitumor activity in multiple myeloma and acute lymphoblastic leukemia, respectively. However, BCMA and CD22, like CD19, are highly restricted to the B cell lineage, which resides in tissue that can be targeted with manageable toxicity. Attempts to target tumor-associated antigens in solid tumors have achieved limited success so far.

The difficulty with solid tumors is that they are usually surrounded by a hostile, immunosuppressive microenvironment. This environment presents many inhibitory factors that prevent CAR-T cells from reaching them. A typical CAR-T approach will not have success under these conditions. For this reason, CAR-T cells with novel designs are in development which are engineered to express additional cytokines to promote CAR functions, such as interkeulin-12 (IL-12) (Chmielewski, M. and H. Abken (2017) Cell Rep 21(11): 3205-3219; Pegram, H. J., et al. (2012) Blood 119(18): 4133-4141; Zhang, T., et al. (2015) ONCOTARGET 6(32): 33961-33971), IL-15 (Hurton, L. V., et al. (2016) Proc Natl Acad Sci U S A 113(48): E7788-e7797), and interferon-β (IFN-β) (Zhao, Z., et al. (2015) Cancer Cell 28(4): 415-428).

The group of Carl H. June (Hu, B., et al. (2017) Cell Reports 20(13): 3025-3033) hypothesized that synthetic IL-18 expression might promote CAR-T cell functions. IL-18 was initially characterized as an inducer of interferon-γ (IFN-γ) expression in T cells and has been shown to activate lymphocytes and monocytes without eliciting severe dose-limiting toxicity in clinical trials. Hu et al reported potent IL-18-mediated effects on human and mouse T cell proliferation. Further, synthetic IL-18 secretion by CAR-T cells was shown to significantly enhanced CAR-T cell expansion and antitumor activity.

Currently, these cells are in preclinical and early clinical development and show a strong performance in solid tumors.

Although some degree of immune stimulation and inflammation was expected with T cell activation after ACT, severe cytokine release syndrome (CRS) has been observed with CD19-specific, BCMAspecific, and CD22-specific CAR-T cells. The severity of the CAR-T cell-associated CRS correlates in part with tumor burden. In the most severe form, CRS shares many features with hemophagocytic lymphohistiocytosis and macrophage activation syndrome including unremitting fevers, hypotension, hypoxia (lung involvement), other end-organ damage and dysfunction including liver and kidney, cytopenias, coagulopathy, hemophagocytosis and neurologic toxicities (Brudno, J. N. and J. N. Kochenderfer (2019). "Recent advances in CAR T-cell toxicity: Mechanisms, manifestations and management." Blood Rev 34: 45-55).

Although CRS has an expected toxicity with T cell immunotherapy, unexpected neurologic complications ranging in severity from mild to life-threatening have also been reported across different clinical studies with CD19- and BCMA specific CAR-T cells. The neurologic toxicities described with CD19-specific CAR-T cells have been largely reversible. It is not known whether the cerebral edema resulting from CAR-T cell therapy is an extreme manifestation of CRS or whether there is a separate mechanism of action. In support of the latter, there is evidence for endothelial injury, perhaps related to inflammatory cytokines, contributing to the onset of neurotoxicity. The mechanisms underlying T cell immunotherapy-mediated CRS and cerebral edema are poorly understood, in part because the field lacks informative animal models to study these important toxicities.

Several approaches were proposed for the management of these CAR-T side effects. For example, the use of tocilizumab to provide IL-6R blockade has improved the management of CRS symptomatology. Nevertheless, the impact of IL-6R blockade on the neurologic sequela following CD19-specific CAR-T cells is unknown.

Immunosuppression with systemic corticosteroid can also improve the symptoms of CRS, with dexamethasone as a logical first choice agent due to its superior central nervous system penetration. Not surprisingly, the use of prolonged systemic corticosteroids has been shown to diminish the persistence and potentially, the efficacy of CAR-T cells.

There is therefore a need for alternative and more effective management solutions that help avoiding or minimizing these side effects of the CAR-T cell treatment.

It was now surprisingly found within the scope of the present invention that IL-18 binding molecules such as, for example, an IL-18 binding protein (IL-18BP), can be used as a co-medication in CAR-T cell (CAR-T) tumor therapy to avoid or minimize side effects, in particular as a modulator of free IL-18 levels in the patient. Co-medication of the IL-18 binding molecule, in particular the IL-18 binding protein as provided herein, is particularly envisaged subsequently to the CAR T-cell transfer and/or upon clinical manifestation of side effects, e.g. inflammatory reactions, such as an IL-18 driven hyperinflammatory status, e.g. as characterized by Macrophage Activation Syndrome-like manifestations, e.g. such as fever, tachychardia, liver damage, henophagocytosis, hypertriglyceridemia, multilineage cytopenias, coagulopathy central nervous system dysfunction and/or enterocolitis.

The IL-18 binding molecule of the invention can be used as a modulator of IL-18 levels in CAR-T cell therapy using cells engineered to express additional cytokines such as IL-18 to promote CAR functions.

The present invention thus provides new opportunities in cancer therapy.

The therapeutic approach of the invention may be combined with a precise quantification of free IL-18 in body fluids of a patient by using a quantification assay as decribed in WO 2015/032932 and/or WO 2016/139297, respectively, the disclosures of which are incorporated herein by reference.

It is therefore an objective of the present invention to provide means and methods useful to control side effects in CAR-T cell therapy, particularly toxic side effects due to an overshooting of the immune system and/or a cytokine release syndrome.

The technical problem is solved by the embodiments provided herein, in particular as characterized in the claims.

Accordingly, the present invention relates to, inter alia, the following embodiments:
1. An IL-18 binding molecule for use in a Chimeric Antigen Receptor T-cell (CAR-T) therapy of a patient diagnosed with a disease associated with expression of a tumor antigen to avoid or minimize side effects related to the CAR-T therapy.
2. The IL-18 binding molecule for use of embodiment 1 as a modulator of IL-18 levels in the patient to avoid or minimize side effects related to the CAR-T therapy.
3. The IL-18 binding molecule for use of embodiment 1 or 2, wherein the disease associated with expression of a tumor antigen is a proliferative disease, a precancerous condition, a cancer, and/or a non-cancer related indication associated with expression of the tumor antigen.
4. The IL-18 binding molecule for use of any one of embodiments 1 to 3, wherein the patient has cancer, particularly a hematological cancer or a solid tumor, particularly a tumor expressing tumor-associated antigens.
5. The IL-18 binding molecule for use of embodiment 4, wherein the cancer is a hematologic cancer selected from one or more of chronic lymphocytic leukemia (CLL), acute leukemia, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative condition, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, and Waldenstrom macroglobulinemia.
6. The IL-18 binding molecule for use of any one of embodiments 1-5, wherein a population of cells is used in the CAR-T therapy, which are engineered to express a chimeric antigen receptor (CAR), wherein the CAR comprises an antigen-binding domain, a transmembrane domain, and an intracellular signaling domain.
7. The IL-18 binding molecule for use of embodiment 6, wherein the cells are further engineered to express IL-18.
8. The IL-18 binding molecule for use of any one of embodiments 1 to 7, wherein the side effects are due to an overshooting of the immune system and/or a cytokine release syndrome.
9. The IL-18 binding molecule for use of embodiment 8, wherein the side effects are selected from the group consisting of unremitting fever, hypotension, hypoxia (lung involvement), other end-organ damage and dysfunction including but not limited to liver and kidney, cytopenias, coagulopathy, hemophagocytosis and neurologic toxicities.
10. The IL-18 binding molecule for use of any one of embodiments 1 to 9, as a modulator of free IL-18 levels in the patient.
11. The IL-18 binding molecule for use of any one of embodiments 1 to 10 for decreasing the IL-18 levels, particularly the free IL-18 levels, in the patient.
12. The IL-18 binding molecule for use of any one of embodiments 1 to 11, wherein the patient is an animal, particularly a human.
13. The IL-18 binding molecule for use of any one of embodiments 1 to 12, which is an IL-18 binding protein (IL-18BP), including any functional equivalent or functional part thereof which retains the modulator activity.
14. The IL-18 binding molecule for use of any one of embodiments 1 to 12, which is an IL-18 binding antibody that specifically binds to free IL-18, but not to IL-18 bound in a complex, including any functional equivalent or functional part thereof which retains the modulator activity.
15. The IL-18 binding molecule for use of embodiment 13, which is a human IL-18BP (hIL-18 BP), preferably a recombinant human IL-18BP (rhIL-18 BP), including any functional equivalent or functional part thereof, which retains the modulator activity.
16. The IL-18 binding molecule for use of embodiment 15, wherein said human IL-18BP is selected from isoform a, b, c and d of human IL-18BP, particularly isoform a as in SEQ ID NO: 2, isoform b as in SEQ ID NO: 3, isoform c as in SEQ ID NO: 4 or isoform d as in SEQ ID NO: 5, including any functional equivalent or functional part of isoforms a, b, c and/or d, which retains the modulator activity.
17. The IL-18 binding molecule for use of embodiment 16, which is an IL-18BP as shown in SEQ ID NO: 2, including any functional equivalent or functional part thereof, which retains the modulator activity, preferably wherein the functional equivalent has a sequence identity of 80%, 85%, 90%, 91%, 92%, 93%, 94%, 95%, 96%, 97%, 98%, 99% to the sequence depicted in SEQ ID NO: 2 and retains the modulator activity.
18. The IL-18 binding molecule for use of embodiment 17, wherein the functional equivalent or functional part thereof includes a mutein of IL-18BP, a fragment, a peptide, a functional derivative, a functional fragment, a fraction, a circularly permuted derivative, a fused protein comprising IL-18BP, an isoform or a salt thereof which retains the modulator activity.
19. A composition comprising the IL-18 binding molecule, particularly an IL-18 binding molecule of any one of embodiments 13-18, for use in a Chimeric Antigen Receptor T-cell (CAR-T) therapy of a patient, diagnosed with a disease associated with expression of a tumor antigen, to avoid or minimize side effects related to the CAR-T therapy.
20. The composition for use of embodiment 19, as a modulator of IL-18 levels in the patient to avoid or minimize side effects related to the CAR-T therapy.
21. The composition for use of embodiment 19 or 20, wherein the side effects are due to an overshooting of the immune system and/or a cytokine release syndrome.
22. The composition for use of any one of embodiments 19 to 21, wherein the disease is a proliferative disease, a precancerous condition, a cancer, and a non-cancer related indication associated with expression of the tumor antigen.
23. The composition for use of any one of embodiments 19 to 22, wherein the patient has cancer, particularly a hematological cancer or a solid tumor, particularly a tumor expressing tumor-associated antigens.
24. The composition for use of embodiment 23, wherein the cancer is a hematologic cancer selected from one or more of chronic lymphocytic leukemia (CLL), acute leukemia, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative condition, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, and Waldenstrom macroglobulinemia.
25. The composition for use of any one of embodiments 19 to 24, wherein the side effects are selected from the group consisting of unremitting fevers, hypotension, hypoxia (lung involvement), other end-organ damage and dysfunction including but not limited to liver and kidney, cytopenias, coagulopathy, hemophagocytosis and neurologic toxicities.
26. The composition for use of any one of embodiments 19 to 25 comprising
   (i) a population of cells engineered to express a chimeric antigen receptor (CAR), wherein the CAR comprises an antigen-binding domain, a transmembrane domain, and an intracellular signaling domain, and
   (ii) an IL-18 binding molecule, particularly the IL-18 binding molecule of any one of embodiments 13 to 18.
27. The composition for use of embodiment 26, wherein the population of cells of (i) are further engineered to express IL-18.
28. The composition for use of any one of embodiments 19 to 27, wherein the IL-18 binding protein (IL-18BP) further comprises N-terminal and/or C-terminal deletion variants of IL-18BP.
29. The composition for use of any one of embodiments 19 to 28, wherein the patient is an animal, particularly a human.
30. The composition for use of any one of embodiments 19 to 29, comprising N- terminal and/or C-terminal deletion variants of IL-18BP in an amount of up to 40%, particularly up to 30%, particularly up to 20%, particularly up to 15%, particularly up to 10%, particularly up to 7.5%, particularly up to 5%, particularly up to 2.5%, particularly up to 1%, particularly up to 0.5%, particularly up to 0.25%, particularly up to 0.1%, particularly up to 0.05%, particularly up to 0.01%.
31. The composition for use of embodiment 28 or 30, wherein said deletion variants comprise deletions of between 1 and 5 amino acid residues at the C-terminal end of the IL-18BP and/or between 1 and 30 amino acid residues at the N-terminal end of the IL-18BP.
32. The composition for use of any one of embodiments 28, 30 or 31, wherein said N-terminal and/or C-terminal deletion variants of IL-18BP are present in an amount of up to 40%, particularly in an amount of between 2% and 35%.
33. The IL-18 binding molecule of any one of embodiments 1 to 18 or the composition of any one of embodiments 19 to 32 for controlling and/or modulating, particularly decreasing, IL-18 levels, particularly free IL-18 levels, in the patient.
34. A method for modulating IL-18 levels in a patient under a Chimeric Antigen Receptor T-cell (CAR-T) therapy to avoid or minimize side effects, comprising administering to a patient in need thereof a therapeutically effective amount of an IL-18 binding molecule, particularly an IL-18 binding molecule according to any one of embodiments 13 to 18, or a therapeutically effective amount of a composition of any one of embodiments 19 to 32.
35. The method for use of embodiment 34, for modulating, particularly decreasing, IL-18 levels, particularly free IL-18 levels, in the patient.
36. The method for use of embodiment 34 or 35, wherein the patient has a disease associated with expression of a tumor antigen, e.g., a proliferative disease, a precancerous condition, a cancer, and a non-cancer related indication associated with expression of the tumor antigen.
37. The method for use of embodiment 36, wherein the patient has cancer, particularly a hematological cancer or a solid tumor, particularly a tumor expressing tumor-associated antigens.
38. The method for use of embodiment 37, wherein the cancer is a hematologic cancer selected from one or more of chronic lymphocytic leukemia (CLL), acute leukemia, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, and Waldenstrom macroglobulinemia.
39. The method for use of any one of embodiments 34 to 38, wherein the patient is an animal, particularly a human.
40. The method of any one of embodiments 34 to 39, wherein the CAR-T cells and the IL-18 binding molecule or the composition, respectively, are administered to the patient concomitantly.
41. The method of any one of embodiments 34 to 39, wherein the CAR-T cells and the IL-18 binding molecule or the composition, respectively, are administered to the patient sequentially.
42. The method of any one of embodiments 34 to 41, wherein the IL-18 binding molecule or the composition, respectively, is administered to the patient as an acute intervention during the CAR-T therapy.
43. The IL-18 binding molecule for use of embodiment 4 or the composition for use of embodiment 23 or the method of embodiment 37 wherein the cancer is a solid tumor, particularly selected from the group consisting of colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancer, combinations of said cancers, and metastatic lesions of said cancers.
44. The IL-18 binding molecule for use of any one of embodiments 1 to 18, 33 or 43 or the composition for use of any one of embodiments 19 to 33 or 43, or the method of any one of embodiments 34 to 43, wherein in a body fluid of the patient an abnormal level of free IL-18 has been determined, in particular wherein the level of free IL-18 exceeds the level of free IL-18 in body fluids of a healthy control subject, particularly by 5%, 10%, 20%, 30%, 40%, 50%, 60%, 70%, 80%, 90%, 100%, or more than 100%, using an assay capable of detecting free IL-18 in body fluids, said assay comprising the IL-18 binding molecule as defined in any one of embodiments 13 to 18.
45. The IL-18 binding molecule for use, the composition for use or the method of embodiment 44, wherein the assay for quantifying the level of free IL-18 in the body fluid(s) comprises the following steps:
   a) bringing a sample of body fluid suspected to contain free IL-18 into contact with the IL-18 binding molecule as defined in any one of embodiments 13 to 18 as the capturing molecule for free IL-18;
   b) allowing the IL-18 binding molecule to bind to free IL-18; and
   c) detecting the binding of the IL-18 binding molecule and determining the amount of free IL-18 in the sample.
46. The IL-18 binding molecule for use, the composition for use or the method of any one of embodiments 44 or 45, wherein the body fluid is selected from the group consisting of broncho-alveolar lavage fluid (BALF) circulation fluids, secretion fluids, biopsy, and homogenized tissue, particularly serum, urine, tear, saliva, bile, sweat, exhalation or expiration, sputum, bronchoalveolar fluid, sebum, cellular, gland, mucosa and tissue secretion.
47. The IL-18 binding molecule for use of any one of embodiments 1 to 18, 33 or 43 to 46 or the composition for use of any one of embodiments 19 to 33 or 43 to 46, or the method of any one of embodiments 34 to 46, wherein the therapy of the patient further comprises the use of one or more check-point inhibitor(s).
48. The IL-18 binding molecule for use of embodiment 47 or the composition for use of embodiment 47, or the method of embodiment 47, wherein the check-point inhibitor is a CTLA-4 inhibitor, a PD-1 inhibitor or a PD-L1 inhibitor.

Accordingly, the invention relates to an IL-18 binding molecule, particularly the IL-18 binding protein (IL-18BP), for use as a co-medication in CAR-T therapy of a patient, who has a disease associated with expression of a tumor antigen, e.g., a proliferative disease, a precancerous condition, a cancer, and/or a non-cancer related indication associated with expression of the tumor antigen; in particular as a modulator, particularly a modulator of IL-18, particularly a modulator of free IL-18 in body samples and/or the body fluids of the patient under CAR-T therapy, which IL-18 levels may be increased due to the cancer and/or CAR-T cells engineered to secrete IL-18, to avoid or minimize side effects related to the CAR-T therapy.

In a specific embodiment, by using the IL-18 binding molecule of the invention, in particular the IL-18BP of the invention, the IL-18 levels in the body fluids of the patient under CAR-T therapy are modulated, particularly decreased, in order to avoid or minimize side effects related to the CAR-T therapy, but particularly the risk of overshooting immune reactions and/or toxic side effects.

In a specific embodiment of the invention, the side effects are selected from the group consisting of unremitting fever, hypotension, hypoxia (lung involvement), other end-organ damage and dysfunction including but not limited to liver and kidney, cytopenias, coagulopathy, hemophagocytosis and neurologic toxicities

Within the invention, a precise quantification of the free IL-18 level in a body sample and/or body fluids of the patient under CAR-T therapy may be made by using a quantification assay as decribed in WO 2015/032932 and/or WO 2016/139297, respectively. Dependent on the free IL-18 level determined in an assay described therein, the amount of IL-18 binding molecule may be determined that ensures an increase in the efficacy of the CAR-T therapy by avoiding or minimizing side effects, particularly side effects which are due to an overshooting of the immune system and/or a cytokine release syndrome.

Within the present invention, the assay may be used to ensure that only those patients are selected for CAR-T therapy that are in need of such a treatment, e.g., patients that have elevated levels of IL-18, particularly elevated levels of free IL-18, in their body fluids, and which are therefore likely to respond to a co-medication with an IL-18 binding molecule as defined herein, particulary an IL-18 binding protein (IL-18BP) as defined herein.

The assay may additionally or alternatively be used to monitor the efficacy of a CAR-T co-medication with an IL-18 binding molecule as defined herein, particulary an IL-18 binding protein (IL-18BP) as defined herein. As such, a decrease of elevated IL-18 levels, particularly of free IL-18 levels, in the body fluids of the treated patient indicates that the co-medication is efficient.

In particular, the assay that may be used for quantifying the levels of free IL-18 in the body samples and/or body fluids of a patient under CAR-T therapy may comprise the following steps:
a) bringing a body sample or a sample of body fluid suspected to contain free IL-18 into contact with an IL-18 binding molecule, in particular an IL-18 binding molecule as defined herein as the capturing molecule for free IL-18;
b) allowing the IL-18 binding molecule to bind to free IL-18;
c) detecting the binding of the IL-18 binding molecule and determining the amount of free IL-18 in the sample.

Within the present invention, the body samples and/or body fluids may be selected from the group consisting of broncho-alveolar lavage fluid (BALF) circulation fluids, secretion fluids, biopsy, and homogenized tissue, particularly serum, urine, tear, saliva, bile, sweat, exhalation or expiration, sputum, bronchoalveolar fluid, sebum, cellular, gland, mucosa and tissue secretion.

In a specific embodiment, the IL-18 binding molecule of the invention is an antibody that specifically binds to free IL-18, but not to IL-18 bound in a complex.

In another specific embodiment, the IL-18 binding molecule of the invention is an IL-18 binding protein (IL-18BP), particularly human IL-18BP (hIL-18BP), particularly recombinant human IL-18BP (rhIL-18BP), including any functional equivalent or functional part thereof, which retains the modulator activity of the reference molecule.

In another specific embodiment, the IL-18BP is selected from isoform a, b, c and d of IL-18BP, particularly isoform a, particularly isoform c.

In another specific embodiment, the IL-18BP is selected from isoform a, b, c and d IL-18BP as shown in SEQ ID NOs 2, and SEQ ID NOs: 3, 4 and 5, respectively, but especially isoform a of IL-18BP as shown in SEQ ID NO: 2, or isoform c as shown in SEQ ID NO: 4, including any functional equivalent or functional part thereof, which retains the modulator activity of the reference molecule.

Within the present invention, mixtures of the above isoforms in different combinations may be used in the compostion of the invention, but particularly a mixture of isoform a and isoform c, including any functional equivalent or functional part thereof which retains the modulator activity of the reference molecule.

Within the present invention, the term "functional equivalent" or "functional part" is meant to relate to the equivalent or the part of the IL-18 binding reference molecule that has still retained the capability of selectively binding to free IL-18 and thus to function as a modulator in CAR-T therapy to avoid or minimize side effects.

Also comprised within the scope of the present invention is a functional mutein of IL-18BP, a functional fragment, a functional peptide, a functional derivative, a functional fragment, a functional fraction, a functional circularly permuted derivative, a functional fused protein comprising IL-18BP, an functional isoform or a functional salt thereof, which retains the capability of selectively binding to free IL-18 and thus to function in CAR-T therapy to avoid or minimize side effects, in particular to function as a modulator of free IL-18 levels in a patient under CAR-T therapy.

In one embodiment, the invention relates to an IL-18BP, which is a fused protein comprising all or part of an IL-18BP, fused to all or part of an immunoglobulin, preferably to the constant region of an immunoglobulin, and wherein the fused protein is still capable of selectively binding to free IL-18, which fused protein retains the capability of selectively binding to free IL-18 and thus to function in CAR-T therapy to avoid or minimize side effects, in particular to function as a modulator of free IL-18 levels in a patient under CAR-T therapy. More specifically, the immunoglobulin may be of the IgG1 or IgG2 isotype, for use in a composition according to any one of the embodiments described herein.

In another embodiment the invention relates to an IL-18BP, which is a fused protein comprising all or part of an IL-18BP, fused to all or part of a small molecule, particularly to all or part of a small molecular drug, optionally wherein the fusion is through a linker molecule and wherein the fused protein retains the capability of selectively binding to free IL-18 and thus to function in CAR-T therapy to avoid or minimize side effects, in particular to function as a modulator of free IL-18 levels in a patient under CAR-T therapy.

In one embodiment, the present invention provides the IL-18 binding molecule for use of any one of the embodiments described herein, wherein the inhibitor is an IL-18 binding protein (IL-18BP) which has a sequence identity of 80%, 81%, 82%, 83%, 84%, 85%, 86%, 87%, 88%, 89%, 90%, 91%, 92%, 93%, 94%, 95%, 96%, 97%, 98%, 99% or 100% to the sequence depicted in SEQ ID NO: 2 and SEQ ID NOs: 3, 4 and 5, but particularly to the sequence depicted in SEQ ID NO: 2 and SEQ ID NO: 4 and has retained the capability of selectively binding to free IL-18 and thus to function in CAR-T therapy to avoid or minimize side effects, in particular to function as a modulator of free IL-18 levels in a patient under CAR-T therapy.

In another embodiment of the invention, the IL-18BP may be accompanied by N- terminal and/or C-terminal deletion variants of IL-18BP in an amount of up to 40%, particularly up to 30%, particularly up to 20%, particularly up to 15%, particularly up to 10%, particularly up to 7.5%, particularly up to 5%, particularly up to 2.5%, particularly up to 1%, particularly up to 0.5%, particularly up to 0.25%, particularly up to 0.1%, particularly up to 0.05%, particularly up to 0.01%.

In a specific embodiment, said N- terminal and/or C-terminal deletion variants of IL-18BP are present in an amount of up to 40%, particularly in an amount of between 2% and 35%.

In particular, said deletion variants may comprise deletions of between 1 and 5 amino acid residues at the C-terminal end of the IL-18BP and/or between 1 and 30 amino acid residues at the N-terminal end of the IL-18BP.

Thus, in one embodiment, the invention relates to a composition comprising the IL-18 binding molecule, particularly the IL-18 binding protein provided herein, for use in a Chimeric Antigen Receptor T-cell (CAR-T) therapy in a patient, who has a disease associated with expression of a tumor antigen, e.g., a proliferative disease, a precancerous condition, a cancer, and/or a non-cancer related indication associated with expression of the tumor antigen,, in particular to increase efficacy of the treatment and/or as a modulator of IL-18 levels in the treated patient to avoid or minimize side effects, particularly side effects related to the CAR-T therapy, particularly side effects which are due to an overshooting of the immune system and/or a cytokine release syndrome.

In a specific em bodiment of the invention, the side effects are selected from the group consisting of unremitting fever, hypotension, hypoxia (lung involvement), other end-organ damage and dysfunction including but not limited to liver and kidney, cytopenias, coagulopathy, hemophagocytosis and neurologic toxicities.

In another embodiment, the invention relates to a composition comprising an IL-18 binding molecule, particularly the IL-18 binding protein provided herein and, in addition, N- terminal and/or C-terminal deletion variants of the IL-18BP provided herein, for use in a Chimeric Antigen Receptor T-cell (CAR-T) therapy in a patient in need thereof to increase efficacy of the treatment and/or for controlling, modulating, particularly decreasing, IL-18 levels, particularly free IL-18 levels, in the patient, particularly throughout the CAR-T therapy.

In particular, the IL-18 binding molecule and the compositon comprising the IL-18 binding molecule according to the present invention may be used as a modulator, particularly a modulator of IL-18 levels in the treated patient, particularly a modulator of free IL-18 levels in the treated patient, to avoid or minimize side effects, particularly side effects related the the CAR-T therapy, particularly side effects which are due to an overshooting of the immune system and/or a cytokine release syndrome.

In particular, the composition of the invention may comprise
(i) a population of cells engineered to express a chimeric antigen receptor (CAR), wherein the CAR comprises an antigen-binding domain, a transmembrane domain, and an intracellular signaling domain, and
(ii) an IL-18 binding molecule, particularly the IL-18 binding protein provided herein.

In a specific embodiment, the population of cells of (i) are further engineered to express IL-18.

In another specific embodiment, the composition further comprises N- terminal and/or C-terminal deletion variants of IL-18BP, particularly N- terminal and/or C-terminal deletion variants of IL-18BP, in particular deletion variants as defined herein.

In another specific embodiment, the above compositions further comprise a pharmaceutically acceptable carrier and/or excipient.

In various embodiments of the invention, the IL-18 binding molecule, particularly the IL-18BP of the invention as described herein, including any functional equivalent or functional part thereof, or the composition of the invention as described herein, comprising said IL-18 binding molecule, particularly the IL-18BP of the invention and, optionally, further comprising N- and C-terminal deletion variants of the IL-18BP, including any functional equivalent or functional part thereof, is used as a co-medication in CAR-T therapy, in particular as a modulator, to avoid or minimize side effects, particularly side effects which are due to an overshooting of the immune system and/or a cytokine release syndrome.

In particular, the CAR-T therapy is used in a patient, who has a disease associated with expression of a tumor antigen, e.g., a proliferative disease, a precancerous condition, a cancer, and/or a non-cancer related indication associated with expression of the tumor antigen.

In particular, the IL-18 binding molecule and the composition comprising the IL-18 binding molecule of the invention is used in CAR-T therapy in a mammal, particularly in a human.

In a specific embodiment, the IL-18 binding molecule and the composition comprising the IL-18 binding molecule of the invention is used in CAR-T therapy, particularly for treating hematological tumors and/or solid tumors, particularly tumors expressing tumor-associated antigens.

In particular, the cancer may be a hematologic cancer, particularly a hematological cancer selected from one or more of chronic lymphocytic leukemia (CLL), acute leukemias, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, and preleukemia.

In a specific embodiment, the cancer may be a solid tumor, particularly a cancer selected from the group consisting of colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.

In one embodiment, the IL-18 binding molecule and the composition comprising the IL-18 binding molecule of the invention, particular the composition of the invention comprising (i) a population of cells engineered to express a chimeric antigen receptor (CAR), wherein the CAR comprises an antigen-binding domain, a transmembrane domain, and an intracellular signaling domain and further to express IL-18, and (ii) an IL-18 binding molecule, particularly the IL-18 binding protein provided herein, is used in CAR-T therapy for treating solid tumors, particularly tumors expressing tumor-asscodiated antigens, particularly non-small cell lung cancer, malignant pleuran medothelioma, or multiform glioblastoma.

Therapy or treatment as used herein comprises prevention, halting, alleviation and/or reversion of symptoms associated with the disease or disorder to be treated.

Within the present invention, the therapy or treatment may be continued at least until the treated patient shows a therapeutic response.

In various further embodiments, the invention relates to the IL-18 binding molecule of the invention, particularly the IL-18BP of the invention as defined herein, or the composition comprising the IL-18 binding molecule of the invention, particularly the IL-18BP of the invention as defined herein, for use according to any one of the preceding embodiments, wherein
- increased expression of IFNγ, IL-13 and/or IL-17A is modified, particularly inhibited, compared to untreated subjects suffering from said disease or disorder; and/or
- binding of free IL-18 by the IL-18BP compensates the IL-18/IL-18BP imbalance by trapping and neutralizing the excess of free IL-18 in tissue and circulation; and/or
- IL-18 binding is restricted or inhibited, particularly binding of free IL-18 to IL-18 receptor (IL-18R), but especially free IL-18 binding to IL-18Ra; and/or
- the IL-18BP reduces binding of IL-18 to IL-18 receptor, particularly binding to IL-18Ra by at least 5%, particularly by at least 10%, particularly by at least 15%, particularly by at least 20%, particularly by at least 25%, particularly by at least 30%, particularly by at least 40%, particularly by at least 45%, particularly by at least 50%, particularly by at least 55%, particularly by at least 60%, particularly by at least 65%, particularly by at least 70, particularly by at least 75, particularly by at least 80, particularly by at least 85%, particularly by at least 90%, particularly by at least 95%, particularly by 100%; and/or
- the IL-18BP neutralizes free IL-18 by restricting or preventing IL-18 binding to IL-18 receptor (IL-18R), especially free IL-18 binding to IL-18Ra.

In one embodiment, the invention relates to the IL-18 binding molecule, particularly the IL-18BP, of the invention, including any functional equivalent or functional part thereof, or to the composition comprising the IL-18 binding molecule, particularly the IL-18BP, of the invention, including any functional equivalent or functional part thereof, for use according to any one of the preceding embodiments, wherein the level of free IL-18 in the body fluid(s) has been determined to be ≥5 pg/mL and, particularly, up to 10000 pg/mL as compared to ≤4 pg/mL in the healthy control.

In a specific embodiment, the level of free IL-18 in the body fluid(s) is two to three times above normal.

The determination of free IL-18 in the body fluid(s) may be accomplished by using an assay for quantifying the level of free IL-18 in the body fluids, in a body sample or *in situ,* which includes the steps of:
a) bringing a sample of body fluid or a body sample or body area suspected to contain free IL-18 into contact with an IL-18 binding molecule, particularly IL-18BP, which specifically binds to free IL-18, but not to IL-18 bound in a complex and functions as the capturing molecule for free IL-18;
b) allowing the IL-18 binding molecule, particularly the IL-18BP to bind to free IL-18;
c) detecting the binding of IL-18 to the IL-18 binding molecule, particularly the IL-18BP and determining the amount of free IL-18 in the sample or in situ.

In one embodiment of the invention, the sample is selected from the group consisting of broncho-alveolar lavage fluid (BALF) circulation fluids, secretion fluids, biopsy, and homogenized tissue, particularly serum, urine, tear, saliva, bile, sweat, exhalation or expiration, sputum, bronchoalveolar fluid, sebum, cellular, gland, mucosa and tissue secretion.

For determining the presence or absence of free IL-18 in a sample according to the present invention, any immunoassay format known to those of ordinary skill in the art may be used such as, for example, assay formats which utilize indirect detection methods using secondary reagents for detection, in particular, ELISA's and immunoprecipitation and agglutination assays may be used. A detailed description of these assays is, for example, given in Harlow and Lane, Antibodies: A Laboratory Manual (Cold Spring Harbor Laboratory, New York 1988 555-612, WO96/13590 to Maertens and Stuyver, Zrein et al. (1998) and WO96/29605.

The sample may be a non-diluted or diluted biological fluid, such as, without being restricted thereto, serum, urine, tear, saliva, bile, sweat, exhalation or expiration, sputum, bronchoalveolar fluid, sebum, cellular, gland, mucosa or tissue secretion, biopsy, homogenized tissue.

For *in situ* diagnosis, the IL-18 binding molecule, particularly the IL-18BP or any active and functional part thereof may be administered to the subject to be diagnosed by methods known in the art such as, for example, intravenous, intranasal, intraperitoneal, intracerebral, intraarterial injection such that a specific binding between the IL-18BP with free IL-18 may occur. The IL-18 binding molecule, particularly the IL-18BP, or any active and functional part thereof may comprise a detectable label, as described further below.

In another aspect of the invention, detection of free IL-18 described herein may be accomplished by an immunoassay procedure. The immunoassay typically includes contacting a test sample with an IL-18 binding molecule, particularly the IL-18BP as described herein in the various embodiments that specifically binds to free IL-18 and detecting the presence of the the IL-18BP/free IL-18 complex in the sample. The immunoassay procedure may be selected from a wide variety of immunoassay procedures known to those skilled in the art such as, for example, competitive or non-competitive enzyme-based immunoassays, enzyme-linked immunosorbent assays (ELISA), radioimmunoassay (RIA), and Western blots, etc. Further, multiplex assays may be used, including arrays, wherein the IL-18 binding molecule, particularly the IL-18BP are placed on a support, such as a glass bead or plate, and reacted or otherwise contacted with the test sample.

Various chemical or biochemical derivatives of the IL-18 binding molecule, in particular the IL-18BP can be produced using known methods. One type of derivative which is diagnostically useful as an immunoconjugate comprising an IL-18 molecule, in particular IL-18BP is conjugated to a detectable label. However, in many embodiments, the IL-18 molecule, in particular IL-18BP is not labeled but in the course of an assay, it becomes indirectly labeled by binding to or being bound by another molecule that is labeled. The invention encompasses molecular complexes comprising an IL-18 molecule, in particular IL-18BP and a label.

Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferones, fluoresceins, fluorescein isothiocyanate, rhodamines, dichlorotriazinylamine fluorescein, dansyl chloride, phycoerythrins, Alexa Fluor 647, Alexa Fluor 680, DiIC₁₉(3), Rhodamine Red-X, Alexa Fluor 660, Alexa Fluor 546, Texas Red, YOYO-1 + DNA, tetramethylrhodamine, Alexa Fluor 594, BODIPY FL, Alexa Fluor 488, Fluorescein, BODIPY TR, BODIPY TMR, carboxy SNARF-1, FM 1-43, Fura-2, Indo-1, Cascade Blue, NBD, DAPI, Alexa Fluor 350, aminomethylcoumarin, Lucifer yellow, Propidium iodide, or dansylamide; an example of a luminescent material includes luminol; examples of bioluminescent materials include green fluorescent proteins, modified green fluorescent proteins, luciferase, luciferin, and aequorin, and examples of suitable radioactive material include ¹²⁵I, ¹³¹I, ³⁵S or ³H.

The immunoassays will typically comprise incubating a sample, such as a biological fluid, a tissue extract, freshly harvested cells, or lysates of cells, in the presence of a detectably labeled IL-18 molecule, in particular IL-18BP, and detecting the bound IL-18 molecule, in particular IL-18BP by any of a number of techniques well-known in the art. One way of measuring the level of free IL-18 with the IL-18BP is by enzyme immunoassay (EIA) such as an enzyme-linked immunosorbent assay (ELISA) (Voller, A. et al., J. Clin. Pathol. 31:507-520 (1978); Butler, J.E., Meth. Enzymol. 73:482-523 (1981); Maggio, E. (ed.), Enzyme Immunoassay, CRC Press, Boca Raton, FL, 1980). The enzyme, either conjugated to the IL-18 molecule, in particular IL-18BP or to a binding partner for the IL-18 molecule, in particular IL-18BP, when later exposed to an appropriate substrate, will react with the substrate in such a manner as to produce a chemical moiety which can be detected, for example, by spectrophotometric, or fluorimetric means.

In a specific embodiment of the invention, the IL-18 molecule, in particular IL-18BP used in any of the above formats, but particularly in an ELISA format is IL-18BP isoform a, b, c or d, or a functional equivalent or a functional derivate thereof, or a functional fragment thereof, particularly isoform a, particularly isoform c, or a derivate thereof, particularly isoform a, b, c or d as shown in SEQ ID NOs 2, and 3, 4 and 5, but especially the isoform a of IL-18BP as shown in SEQ ID NO: 2 or the isoform c as shown in SEQ ID NO 4.

Also, mixtures of the above isoforms may be used in the composition of the invention, but particularly a mixture of isoform a and isoform c.

The biological sample may be brought in contact with and immobilized onto a solid phase support or carrier such as nitrocellulose, or other solid support which is capable of immobilizing cells, cell particles or soluble proteins. The support may then be washed with suitable buffers followed by treatment with the detectably labeled IL-18 binding molecule, particularly IL-18BP. The solid phase support may then be washed with the buffer a second time to remove unbound IL-18 binding molecule, particularly unbound IL-18BP. The amount of bound label on solid support may then be detected by conventional means. A well-known example of such a technique is Western blotting.

In various embodiments, the present invention provides compositions comprising labeled IL-18 molecule, in particular IL-18BP according to the invention as described herein.

In still another embodiment, the invention relates to a method for modulating side effects in a Chimeric Antigen Receptor T-cell (CAR-T) cancer therapy, said method comprising:
a. in a first optional step, quantifying the amount of free IL-18 in the body fluids of said subject using the method as described herein in the various embodiments;
b. administering to a patient in need thereof a therapeutically effective amount of an IL-18 binding molecule, particularly an IL-18 binding protein as provided herein, or an effective amount of a composition as provided herein.

In particular, a patient in need of such a treatment is a patient, whose level of free IL-18 has been determined in a body sample or in the body fluids to exceed the level of free IL-18 in a body sample or the body fluids of a healthy control subject by 5%, 10%, 20%, 30%, 40%, 50%, 60%, 70%, 80%, 90%, 100%, or more than 100%.

A therapeutically or prophylactically effective amount of an IL-18 binding molecule or a composition comprising said IL-18 binding molecule as defined in any one of the preceeding embodiments, may be administered to the patient by systemic, intranasal, buccal, oral, transmucosal, intratracheal, intravenous, subcutaneous, intraurinary tract, intravaginal, sublingual, intrabronchial, intrapulmonary, transdermal or intramuscular administration, in particular broncho-pulmonary administration.

The IL-18 binding molecule or the composition comprising the IL-18 binding molecule of the invention and as disclosed herein in the various embodiments is administered to a patient in need thereof in a suitable dosage form and/or unit and/or dosage interval.

In a specific embodiment, the IL-18 binding molecule, particularly the IL-18BP, of the invention, including any functional equivalent or functional part thereof, as defined herein, is formulated as a pharmaceutical composition, in particular a pharmaceutical composition comprising a sterile solution for injection, preferably further sodium chloride, and/or sodium hydroxide and/or sodium phosphate buffer, particularly in a concentration of between 0.01 M and 0.2 M, particularly between 0.01 M and 0.15 M. It is preferred herein to include sodium chloride and sodium phosphate buffer in the composition of the invention. In a preferred embodiment, the composition of the invention, in particular the pharmaceutical composition, comprises about 0.12 M sodium chloride and about 0.02 M sodium phosphate buffer.

The IL-18 binding molecule, particularly the IL-18BP, of the invention, including any functional equivalent or functional part thereof, or to the composition comprising the IL-18 binding molecule, particularly the IL-18BP of the invention, including any functional equivalent or functional part thereof, may be administered to a patient in need thereof in a single dose or dosage unit/day, in multiple doses or doseage units/day, in multiple doses or doseage units /week or in multiple doses or doseage units /month. The single dose or dosage unit may be split into several doses or dosage units and administered to the patient to be treated over several hours or a whole day.

In one embodiment, the IL-18 binding molecule, particularly the IL-18BP, of the invention, including any functional equivalent or functional part thereof, or to the composition comprising the IL-18 binding molecule, particularly the IL-18BP of the invention, including any functional equivalent or functional part thereof, is administered every 48 hours.

In one embodiment, the IL-18 binding molecule, particularly the IL-18BP, of the invention, including any functional equivalent or functional part thereof, or the composition according to the invention may be administered by s.c. injection. In particular, the site of the s.c. injection is alternated, particularly the site of injection is outside of the thighs and the various quadrants of the anterior abdominal wall. The separate injections that constitute a single dosage of the composition of the invention is particularly administered within the same body region but not at the exact same injection site.

In one embodiment, the IL-18 binding molecule, particularly the IL-18BP, of the invention, including any functional equivalent or functional part thereof, or the composition according to the invention may be administered intravenously. The formulation of the IL-18 binding molecule, particularly the IL-18BP, of the invention, including any functional equivalent or functional part thereof, or the composition according to the invention may be adapted for intravenous administration using methods known in the art.

In one embodiment, the composition is brought to room temperature, particularly between 18 - 25°C, before administration.

In a specific embodiment, a single dose of the composition of the invention and particularly the composition for use according to any one of the preceding embodiments comprises between 10 mg and 600 mg IL-18BP.

In particular, the single dose may comprise between 10 and 20 mg, between 20 and 40 mg, between 40 and 80 mg, between 80 and 160 mg, between 160 mg and 320 mg or between 320 mg and 600 mg IL-18BP.

In various embodiments of the invention, a single dose may comprise between 0.5 mg of IL-18 binding molecule/kg body weight and 10 mg IL-18 binding molecule /kg body weight, particularly between 1 mg IL-18 binding molecule /kg body weight and 8 mg IL-18 binding molecule /kg body weight, particularly between 1,5 mg IL-18 binding molecule /kg body weight and 6 mg IL-18 binding molecule /kg body weight, particularly between 2 mg IL-18 binding molecule /kg body weight and 4 mg IL-18 binding molecule /kg body weight.

In a specific embodiment of the invention, a single dose may comprise between 2 mg of IL-18BP/kg body weight and 3 mg IL-18BP /kg body weight, particularly 2 mg of IL-18BP/kg body weight.

In a specific embodiment, the IL-18 binding molecule, particularly the IL-18BP, of the invention, including any functional equivalent or functional part thereof, or the composition comprising the IL-18 binding molecule, particularly the IL-18BP, of the invention, including any functional equivalent or functional part thereof is administered in a dosage of between 0.5 mg IL-18 binding molecule /kg body weight and 5 mg IL-18 binding molecule /kg body weight every 24 or 48 h, particularly, a single dose of 2 mg IL-18 binding molecule /kg body weight is administered every 48 h.

In another specific embodiment, human IL-18BP (rhIL-18 BP), including any functional equivalent or functional part thereof or the composition comprising a recombinant human IL-18BP (rhIL-18 BP), including any functional equivalent or functional part thereof, is administered in a single dose every other day of 2 mg/kg body weight, for example over three weeks. However, it is preferred that the IL-18 binding molecule, in particular the IL-18 binding protein as provided herein, is administered depending on free IL-18 levels assumed or determined in a patient, in particular that the IL-18 molecule, IL-18 binding protein, is administered until free IL-18 levels are assumed or determined to be below a level that is thought to be acceptable, such as for example a level observed in a healthy subject.

The compositions of the invention may comprise additional medicinal agents, pharmaceutical agents, carriers, excipients, buffers, dispersing agents, diluents, and/or co-therapeutic agents such as anti-inflammatory, bronchodilatory, antihistamine, decongestant, anti-tussive drug substances, antiviral and/or immunosuppressant drugs, and/or anti-cancer agents, such as checkpoint inhibitors, and the like, depending on the intended use and application.

In one embodiment of the present invention, the IL-18 binding molecule or the composition comprising the the IL-18 binding molecule of the invention and as disclosed herein in the various embodiments, is administered prophylactically.

In another embodiment of the present invention, the IL-18 binding molecule or the composition comprising the the IL-18 binding molecule of the invention and as disclosed herein in the various embodiments is administered therapeutically.

In one embodiment of the present invention, the IL-18 binding molecule or the composition comprising the the IL-18 binding molecule of the invention and as disclosed herein in the various embodiments is administered to a patient in need thereof by systemic, intranasal, intraocular, intravitral, eye drops, buccal, oral, transmucosal, intratracheal, intravenous, subcutaneous, intraurinary tract, intrarectal, intravaginal, sublingual, intrabronchial, intrapulmonary, transdermal or intramuscular administration. In particular, the administration is done by subcutanous injection.

The IL-18 binding molecule or the composition comprising the IL-18 binding molecule of the invention and as disclosed herein in the various embodiments may be provided as a liquid, liquid spray, microspheres, semisolid, gel, or powder for transmucosal administration, e.g. intranasal, buccal, oral transmucosal, intratracheal, intraurinary tract, intravaginal, sublingual, intrabronchial, intrapulmonary and/or transdermal administration. Further, the composition may be in a solid dosage form for buccal, oral transmucosal and/or sublingual administration. Intranasal, buccal, oral intratracheal, intraurinary tract, intravaginal, transmucosal and sublingual administrations lead to the disintegration of the composition as described herein in an oral cavity at body temperature and optionally may adhere to the body tissue of the oral cavity. Additionally, the composition as disclosed herein further may include one or more excipient, diluent, binder, lubricant, glidant, disintegrant, desensitizing agent, emulsifier, mucosal adhesive, solubilizer, suspension agent, viscosity modifier, ionic tonicity agent, buffer, carrier, surfactant, flavor, or mixture thereof.

In a specific aspect of the present invention, the IL-18 binding molecule or the composition comprising the IL-18 binding molecule is formulated as a parenteral, or intravenous solution, suspension, emulsion, as a tablet, pill, bioadhesive patch, drops, sponge, film, lozenge, hard candy, wafer, sphere, lollipop, disc-shaped structure, suppository or spray.

Transmucosal administration is generally rapid because of the rich vascular supply to the mucosa and the lack of a stratum corneum in the epidermis. Such drug transport typically provides a rapid rise in blood concentrations, and similarly avoids the enterohepatic circulation and immediate destruction by gastric acid or partial first- pass effects of gut wall and hepatic metabolism. Drugs typically need to have prolonged exposure to a mucosal surface for significant drug absorption to occur.

The transmucosal routes can also be more effective than the oral route in that these routes can provide for relatively faster absorption and onset of therapeutic action. Further, the transmucosal routes can be preferred for use in treating patients who have difficulty in swallowing tablets, capsules, or other oral solids, or those who have disease-compromised intestinal absorption. Accordingly, there are many advantages to transmucosal administration of the IL-18 binding molecule, particularly the IL-18BP, or a composition comprising the IL-18 binding molecule, particularly the IL-18BP and a pharmaceutically acceptable carrier and/or excipient.

In either of the intranasal or buccal routes, drug absorption can be delayed or prolonged, or uptake may be almost as rapid as if an intravenous bolus were administered. Because of the high permeability of the rich blood supply, the sublingual route can provide a rapid onset of action.

The intranasal compositions can be administered by any appropriate method according to their form. A composition including microspheres or a powder can be administered using a nasal insufflator device. Examples of these devices are well known to those of skill in the art, and include commercial powder systems such as Fisons Lomudal System. An insufflator produces a finely divided cloud of the dry powder or microspheres. The insufflator is preferably provided with a mechanism to ensure administration of a substantially fixed amount of the composition. The powder or microspheres can be used directly with an insufflator, which is provided with a bottle or container for the powder or microspheres.

Alternatively, the powder or microspheres can be filled into a capsule such as a gelatin capsule, or other single dose device adapted for nasal administration. The insufflator preferably has a mechanism to break open the capsule or other device. Further, the composition can provide an initial rapid release of the active ingredient followed by a sustained release of the active ingredient, for example, by providing more than one type of microsphere or powder. Further, alternative methods suitable for administering a composition to the nasal cavity will be well known by the person of ordinary skill in the art. Any suitable method may be used. For a more detailed description of suitable methods reference is made to EP2112923, EP1635783, EP1648406, EP2112923 (the entire contents of which are incorporated by reference herein).

In one embodiment of the present invention, the IL-18 binding molecule or the composition comprising the IL-18 binding molecule of the invention and as disclosed herein in the various embodiments may be further administered intranasally, i.e. by inhalation and, thus, may be formulated in a form suitable for intranasal administration, i.e. as an aerosol, dry powder formulation or a liquid preparation. Examples of suitable pharmaceutical carriers, excipients and/or diluents are well known in the art and include, but are not limited to, a gum, a starch (e.g. corn starch, pregeletanized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, talc, or mixtures thereof.

Pharmaceutically acceptable carriers for liquid formulation are aqueous or non-aqueous solutions, suspensions, dry powder formulations, emulsions or oils. Examples of non-aqueous solvents are propylene glycol, polyethylene glycol, and injectable organic esters such as ethyl oleate. Examples of oils are those of animal, vegetable, or synthetic origin, for example, peanut oil, soybean oil, olive oil, sunflower oil, fish-liver oil, another marine oil, or a lipid from milk or eggs.

The present invention also relates to transpulmonary administration by inhalation of the IL-18 binding molecule or the composition comprising the IL-18 binding molecule of the invention and as disclosed herein in the various embodiments as a dry powder, gaseous or volatile formulation into systemic circulation via the respiratory tract. Absorption is virtually as rapid as the formulation can be delivered into the alveoli of the lungs, since the alveolar and vascular epithelial membranes are quite permeable, blood flow is abundant and there is a very large surface for adsorption. For instance, aerosols may be delivered from pressure-packaged, metered-dose inhalers (MDIs).

The IL-18 binding molecule or the composition comprising the IL-18 binding molecule of the invention and as disclosed herein in the various embodiments would generally be administered in a mixture with a suitable pharmaceutical excipient, diluent or carrier selected with regard to the chosen means of inhalation and standard pharmaceutical practice.

In another embodiment of the invention, the IL-18 binding molecule, particularly the IL-18BP or the composition provided herein, is formulated as a dry powder, optionally together with at least one particulate pharmaceutically acceptable carrier, which may be one or more materials known as pharmaceutically acceptable carriers, preferably chosen from materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran, mannitol or sorbitol. An especially preferred carrier is lactose, for example lactose monohydrate or anhydrous lactose. The dry powder may be contained as unit doses in capsules of, for example, gelatin or plastic, or in blisters (e.g. of aluminium or plastic), for use in a dry powder inhalation device, which may be a single dose or multiple dose device, preferably in dosage units together with the carrier in amounts to bring the total weight of powder per capsule to from 5 mg to 50 mg. Alternatively, the dry powder may be contained in a reservoir in a multi-dose dry powder inhalation (MDDPI) device adapted to deliver.

Any other therapeutically efficacious route of administration may be used, for example absorption through epithelial or endothelial tissues or by gene therapy wherein a DNA molecule encoding the active agent is administered to the patient (e.g. via an expression vector), which causes the active agent to be expressed and secreted *in vivo.*

The IL-18 binding molecule or the composition comprising the IL-18 binding molecule of the invention and as disclosed herein in the various embodiments may be used for treatment of a cancer as described herein in the various embodiments in human and veterinary medicine for treating humans and animals, including avians, non-human primates, dogs, cats, pigs, goats, sheep, cattle, horses, mice, rats and rabbits.

In a specific embodiment, the present invention provides the IL-18 binding molecule or the composition comprising the IL-18 binding molecule of the invention as disclosed herein in the various embodiments for use in the treatment of cancer as described herein in the various embodiments, wherein the subject is a mammal, in particular wherein the subject is a human.

Aqueous carriers include water, alcoholic/aqueous solutions, emulsions or suspensions, including saline and buffered media such as phosphate buffered saline solutions, water, emulsions, such as oil/water emulsions, various types of wetting agents, sterile solutions etc. Compositions comprising such carriers can be formulated by well known conventional methods. Suitable carriers may comprise any material which, when combined with the biologically active compound of the invention, the compound retains the biological activity.

Efforts have been made in the art to chemically modify the barrier properties of skin to permit the penetration of certain agents, enhance the effectiveness of the agent being delivered, enhance delivery times, reduce the dosages delivered, reduce the side effects from various delivery methods, reduce patient reactions, and so forth.

In this regard, penetration enhancers have been used to increase the permeability of the dermal surface to drugs, and are often proton accepting solvents such as dimethyl sulfoxide (DMSO) and dimethylacetamide. Other penetration enhancers that have been studied and reported as effective include 2-pyrrolidine, N,N-diethyl-m-toluamide (Deet), 1-dodecal-azacycloheptane-2-one, N,N-dimethylformamide, N-methyl-2-pyrrolidine, calcium thioglycolate, hexanol, fatty acids and esters, pyrrolidone derivatives, derivatives of 1,3-dioxanes and 1,3-dioxolanes, 1-N-dodecyl-2-pyrrolidone-5-carboxylic acid, 2-pentyl-2-oxo-pyrrolidineacetic acid, 2-dodecyl-2-oxo-1-pyrrolidineacetic acid, 1-azacycloheptan-2-one-2-dodecylacetic acid, and aminoalcohol derivatives, including derivatives of 1,3-dioxanes, among others.

Preparations for transmucosal administration may include sterile aqueous or non-aqueous solutions, suspensions, dry powder formulations and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Transmucosal vehicles may include sodium chloride solution, Ringer's dextrose, dextrose and sodium chloride, lactated Ringer's, or fixed oils. Preservatives and other additives may also be present including, for example, antimicrobials, anti-oxidants, chelating agents, and inert gases and the like. In addition, the pharmaceutical composition of the present invention might comprise proteinaceous carriers, like, e.g., serum albumin or immunoglobulin, preferably of human origin.

The IL-18 binding molecule or the composition comprising the IL-18 binding molecule of the invention as disclosed herein in the various embodiments may be administered topically to body surfaces and, thus, be formulated in a form suitable for topical administration. Suitable topical formulations include gels, ointments, creams, lotions, drops and the like. For topical administration, the composition of the invention as disclosed herein in the various embodiments is prepared and applied as a solution, suspension, or emulsion in a physiologically acceptable diluent with or without a pharmaceutical carrier.

The IL-18 binding molecule or the composition comprising the IL-18 binding molecule of the invention and as disclosed herein in the various embodiments may also be administered as controlled-release compositions, i.e. compositions in which the active ingredient is released over a period of time after administration. Controlled- or sustained-release compositions include formulation in lipophilic depots (e.g. fatty acids, waxes, oils). In another embodiment, the composition is an immediate-release composition, i.e. a composition in which all the active ingredient is released immediately after administration.

Further examples for suitable formulations are provided in WO 2006/085983, the entire contents of which is herein incorporated by reference. For example, the IL-18 binding molecule or the composition comprising the IL-18 binding molecule of the invention and as disclosed herein in the various embodiments is of the present invention may be provided as liposomal formulations. The technology for forming liposomal suspensions is well known in the art. The lipid layer employed can be of any conventional composition and can either contain cholesterol or can be cholesterol-free. The liposomes can be reduced in size, as through the use of standard sonication and homogenization techniques. Liposomal formulations containing the pharmaceutical composition of the invention as disclosed herein in the various embodiments can be lyophilized to produce a lyophilizate which can be reconstituted with a pharmaceutically acceptable carrier, such as water, to regenerate a liposomal suspension. The pharmaceutical composition of the invention as disclosed herein in the various embodiments can be administered to the subject at a suitable dose. The dosage regimen will be determined by the attending physician and clinical factors. As is well known in the medical arts, dosages for any one subject depend upon many factors, including the subject's size, body surface area, age, the particular compound to be administered, sex, time and route of administration, general health, and other drugs being administered concurrently.

The dosage of the IL-18 binding molecule or the composition comprising the IL-18 binding molecule of the invention as disclosed herein in the various embodiments will depend on the condition being treated, the particular composition used, and other clinical factors such as weight, size and condition of the subject, body surface area, the particular compound or composition to be administered, other drugs being administered concurrently, and the route of administration.

The IL-18 binding molecule or the composition comprising the IL-18 binding molecule of the invention as disclosed herein in the various embodiments may be administered in combination with other biologically active substances and procedures for the treatment of symptoms associated with cancer therapy. The other biologically active substances may be part of the same composition already comprising the composition according to the invention, in form of a mixture, wherein the composition of the invention and the other biologically active substance are intermixed in or with the same pharmaceutically acceptable solvent and/or carrier or may be provided separately as part of a separate compositions, which may be offered separately or together in form of a kit of parts. Within the present invention, the other biologically active substance may, for example, be a checkpoint inhibitor, such as a CTLA-4, PD-1 or PD-L1 inhibitor.

The IL-18 binding molecule or the composition comprising the IL-18 binding molecule of the invention as disclosed herein in the various embodiments may be administered concomitantly with the other biologically active substance or substances, intermittently or sequentially. For example, the composition according to the invention may be administered simultaneously with a first additional biologically active substance or sequentially after or before administration of said composition. If an application scheme is chosen where more than one additional biologically active substance is administered and at least one composition according to the invention, the compounds or substances may be partially administered simultaneously, partially sequentially in various combinations.

The present invention thus provides for mixtures of the IL-18 binding molecule or the composition comprising the IL-18 binding molecule of the invention as disclosed herein in the various embodiments, optionally comprising, one or more further biologically active substances in a therapeutically or prophylactically effective amount, as well as to methods of using such a mixture for prevention and/or therapeutic treatment.

The other biologically active substance or compound may exert its biological effect by the same or a similar mechanism as the composition according to the invention or by an unrelated mechanism of action or by a multiplicity of related and/or unrelated mechanisms of action.

Suitable dosages of the composition of the invention as disclosed herein in the various embodiments will vary depending upon the condition, age and species of the subject, and can be readily determined by those skilled in the art. The total daily dosages of the employed in both veterinary and human medicine will suitably be in the range of 0.1 to 10 mg per kilogram.

Further, functional derivatives of the IL-18 binding molecule, particularly the IL-18BP, may be conjugated to polymers in order to improve the properties of the protein, such as the stability, half-life, bioavailability, tolerance by the human body, or immunogenicity. To achieve this goal, IL1-8BP may be linked e.g. to Polyethlyenglycol (PEG). PEGylation may be carried out by known methods, described in WO 92/13095, for example.

Therefore, in another embodiment of the present invention, IL-18BP is PEGylated.

In still another embodiment of the invention, IL-18BP is a fused protein comprising all or part of an IL-18BP, which is fused to all or part of an immunoglobulin, preferably to the constant region (Fc) of an immunoglobulin, and wherein the fused protein is still capable of binding to IL-18. More specifically, the immunoglobulin may be of the IgG1 or IgG2 isotype.

In a further embodiment of the invention, the IL-18BP is PEGylated, fused to all or part of an immunoglobulin, preferably to the constant region (Fc) of an immunoglobulin, and wherein the fused protein is still capable of binding to IL-18. More specifically, the immunoglobulin may be of the IgG1 or IgG2 isotype.

The person skilled in the art will understand that the resulting fusion protein retains the biological activity of IL-18BP, in particular the binding to IL-18. The fusion may be direct, or via a short linker peptide which can be as short as 1 to 3 amino acid residues in length or longer, for example, 13 amino acid residues in length. Said linker may be a tripeptide of the sequence E-F-M (Glu-Phe-Met), for example, or a 13-amino acid linker sequence comprising Glu-Phe-Gly-Ala-Gly-Leu-Val-Leu-Gly-Gly-Gln-Phe-Met (SEQ ID NO: 1) introduced between the IL-18BP sequence and the immunoglobulin sequence. The resulting fusion protein has improved properties, such as an extended residence time in body fluids (half-life), increased specific activity, increased expression level, or the purification of the fusion protein is facilitated.

Preferably, it is fused to heavy chain regions, like the CH2 and CH3 domains of human IgG1, for example. The generation of specific fusion proteins comprising IL-18BP and a portion of an immunoglobulin are described in example 11 of WO99/09063, for example. Other isoforms of Ig molecules are also suitable for the generation of fusion proteins according to the present invention, such as isoforms IgG2 or IgG4, or other Ig classes, like IgM or IgA, for example. Fusion proteins may be monomeric or multimeric, hetero or homomultimeric.

### DEFINITIONS

The technical terms and expressions used within the scope of this application are generally to be given the meaning commonly applied to them in the pertinent art if not otherwise indicated herein below.

As used in this specification and the appended embodiments, the singular forms "a", "an", and "the" include plural referents unless the context clearly dictates otherwise. Thus, for example, reference to "a compound" includes one or more compounds.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effects attributed to the disease. The term "treatment" as used herein covers any treatment of a disease in a subject and includes: (a) preventing a disease, i.e. related to an undesired immune response from occurring in a subject which may be predisposed to the disease; (b) inhibiting the disease, i.e. arresting its development; or (c) relieving the disease, i.e. causing regression of the disease (d) reversing the disease symptoms, i.e. leading to recovery of damaged tissue.

The expression "IL-18 Binding Protein (IL-18BP)" as used herein includes the full-length protein, as well as a mutein, fragment, peptide, functional derivative, functional fragment, fraction, circularly permuted derivative, fused protein comprising IL-18BP, isoform or a salt thereof, which have retained the specific activity of the full-length reference protein, particulary the CAR-T modulating activity as described herein.

The term "free IL-18" as used herein means monomeric, soluble and non-complexed interleukin-18 protein.

The term "functional" and "active" are used herein synonymously and refers to a modified IL-18 binding molecule, particularly a modified IL-18BP, or to a part thereof, which still has the same or essentially the same biological, pharmacological and therapeutic properties as the unmodified or full length IL-18 binding molecule, particularly the unmodified IL-18BP, and can thus be used within the present invention for the treatment of the diseases and disorders as disclosed herein the same way as the unmodified or full length IL-18 binding molecule, particularly as the unmodified IL-18BP. In particular, by "functional" is meant that the modified IL-18 binding molecule, particularly the IL-18BP, has still retained the IL-18 moduating activity of the IL-18 binding molecule, particularly the IL-18BP, in CAR-T therapy and is thus still capable of minimizing or avoiding side effects of the CAR-T therapy, particularly by controlling or decresing the levels of free IL-18 in the patients under therapy.

In various embodiments of the invention, the term "IL-18BP" refers to human IL-18BP, particularly to recombinant human IL-18BP, particularly to isoform a, b, c or d of IL-18BP, particularly isoform a, particularly isoform c, particularly isoform a, b, c or d as shown in SEQ ID NOs 2, and SEQ ID NOs: 3, 4 and 5, but especially the isoform a of IL-18BP as shown in SEQ ID NO: 2 or the isoform c as shown in SEQ ID NO 4.

A "patient" or "subject" for the purposes of the present invention is used interchangeably and meant to include both humans and other animals, particularly mammals, and other organisms. Thus, the methods are applicable to both human therapy and veterinary applications. In the preferred embodiment the patient or subject is a mammal, and in the most preferred embodiment the patient or subject is a human.

The expressions "composition" and "pharmaceutical composition" are used herein interchangeably and are meant to refer, for the purposes of the present invention, to a therapeutically effective composition in particular a therapeutically effective amount of the active ingredient, i.e. the IL-18 binding molecule, in particular the IL-18BP and, optionally, a pharmaceutically acceptable carrier and/or excipient.

It embraces compositions that are suitable for the curative treatment, the control, the amelioration, an improvement of the condition or the prevention of a disease or disorder in a human being or a non-human animal. Thus, it embraces pharmaceutical compositions for the use in the area of human or veterinary medicine. Such a "composition" is characterized in that it embraces at least one IL-18 binding molecule, in particular IL-18BP or a physiologically acceptable salt thereof, and optionally a carrier or excipient whereby the salt and the carrier and excipient are tolerated by the target organism that is treated therewith.

A "therapeutically effective amount" refers to that amount which provides a therapeutic effect for a given condition and administration regimen. In particular, "therapeutically effective amount" means an amount that is effective to prevent, reverse, alleviate or ameliorate symptoms of the disease or prolong the survival of the subject being treated, which may be a human or non-human animal. Determination of a therapeutically effective amount is within the skill of the person skilled in the art. In particular, in the present case a "therapeutically or prophylactically effective amount" refers to the amount of protein or peptide, mutein, functional derivative, fraction, circularly permuted derivative, fused protein, isoform or a salt thereof, and compound or pharmaceutical composition which, when administered to a human or animal, leads to a therapeutic or prophylactic effect in said human or animal. The effective amount is readily determined by one of skill in the art following routine procedures. The therapeutically effective amount or dosage of a compound according to this invention can vary within wide limits and may be determined in a manner known in the relevant art. The dosage can vary within wide limits and will, of course, have to be adjusted to the individual requirements in each particular case.

The term "transmucosal" administration refers to various administration routes wherein the compound is absorbed by the mucosa of any part of the body. Transmucosal administration comprises, but is not limited to, i.e. intranasal, buccal, oral transmucosal, intratracheal, intraurinary tract, intrarectal, intravaginal, sublingual, intrabronchial, intrapulmonary and transdermal administration.

The definition "pharmaceutically acceptable" is meant to encompass any carrier, excipient, diluent or vehicle, which does not interfere with effectiveness of the biological activity of the active ingredient and that is not toxic to the host to which it is administered.

The term "fused protein" refers to a polypeptide comprising an IL-18BP, or a viral IL-18BP, or a mutein or fragment thereof, fused with another protein, which, e. g., has an extended residence time in body fluids. An IL-18BP or a viral IL-18BP may thus be fused to another protein, polypeptide or the like, e. g., an immunoglobulin or a fragment thereof.

These isoforms, muteins, fused proteins or functional derivatives retain the biological activity of IL-18BP, in particular the binding to IL-18, and preferably have essentially at least an activity similar to IL-18BP. Ideally, such proteins have a biological activity which is even increased in comparison to unmodified IL-18BP. Preferred active fractions have an activity which is better than the activity of IL-18BP, or which have further advantages, like a better stability or a lower toxicity or immunogenicity, or they are easier to produce in large quantities, or easier to purify.

The term "interleukin-18 binding protein" (IL-18BP) comprises also functional equivalents of the IL-18BP, including mutein, functional derivative, fraction, biologically active peptide, circularly permuted derivative, fused protein, isoform and a salt thereof.

In particular, the functional equivalent of the IL-18BP may have a sequence identity of 80%, 85%, 90%, 91%, 92%, 93%, 94%, 95%, 96%, 97%, 98%, 99% to the sequence depicted in SEQ ID NO: 2 and which retains the modulator activity of the reference molecule, preferably which retains the IL-18 binding specificity and the ability to avoid or minimize side effects as described herein.

As used herein the term "muteins" refers to analogs of an IL-18BP, or analogs of a viral IL-18BP, in which one or more of the amino acid residues of a natural IL-18BP or viral IL-18BP are replaced by different amino acid residues, or are deleted, or one or more amino acid residues are added to the natural sequence of an IL-18BP, or a viral IL-18BP, without changing considerably the activity of the resulting products as compared with the wild type IL-18BP or viral IL-18BP. These muteins are prepared by known synthesis and/or by site-directed mutagenesis techniques, high throughput mutagenesis, DNA shuffling, protein evolution techniques, or any other known technique suitable therefore.

Any such mutein preferably has a sequence of amino acids sufficiently duplicative of that of an IL-18BP, or sufficiently duplicative of a viral IL-18BP, such as to have substantially similar activity to IL-18BP. One activity of IL-18BP is its capability of binding IL-18. As long as the mutein has substantial binding activity to IL-18, it can be used in the purification of IL-18, such as by means of affinity chromatography, and thus can be considered to have substantially similar activity to IL-18BP. Thus, it can be determined whether any given mutein has substantially the same activity as IL-18BP by means of routine experimentation comprising subjecting such a mutein, e. g. to a simple sandwich competition assay to determine whether or not it binds to an appropriately labeled IL-18, such as radioimmunoassay or ELISA assay.

Muteins of IL-18BP polypeptides or muteins of viral IL-18BPs, which can be used in accordance with the present invention, or nucleic acid coding therefore, include a finite set of substantially corresponding sequences as substitution peptides or polynucleotides which can be routinely obtained by one of ordinary skill in the art, without undue experimentation, based on the teachings and guidance presented herein.

Preferred changes for muteins in accordance with the present invention are what are known as "conservative" substitutions. Conservative amino acid substitutions of IL-18BP polypeptides or proteins or viral IL-18BPs, may include synonymous amino acids within a group which have sufficiently similar physicochemical properties that substitution between members of the group will preserve the biological function of the molecule (Grantham, 1974). It is clear that insertions and deletions of amino acids may also be made in the above-defined sequences without altering their function, particularly if the insertions or deletions only involve a few amino acids, e. g. under thirty, and preferably under ten, and do not remove or displace amino acids which are critical to a functional conformation, e. g., cysteine residues. Proteins and muteins produced by such deletions and/or insertions come within the purview of the present invention.

"Functional derivatives" as used herein cover derivatives of IL-18BPs or a viral IL-18BP, and their muteins and fused proteins, which may be prepared from the functional groups which occur as side chains on the residues or the N-or C-terminal groups, by means known in the art, and are included in the invention as long as they remain pharmaceutically acceptable, i. e. they do-not destroy the activity of the protein which is substantially similar to the activity of IL-18BP, or viral IL-18BPs, and do not confer toxic properties on compositions containing it.

These derivatives may, for example, include polyethylene glycol side-chains, which may mask antigen sites and extend the residence of an IL-18BP or a viral IL-18BP in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e. g. alkanol or carbocyclic aroyl groups) or O-acyl derivatives of free hydroxyl groups (for example that of seryl or threonyl residues) formed with acyl moieties.

As "functional fragment" of an IL-18BP, or a viral IL-18BP, mutein and fused protein, the present invention covers any fragment or precursors of the polypeptide chain of the IL-18BP protein molecule alone or together with associated molecules or residues linked thereto, e. g., sugar or phosphate residues, or aggregates of the protein molecule or the sugar residues by themselves, provided said fraction has substantially similar activity to IL-18BP.

The term "salts" herein refers to both salts of carboxyl groups and to acid addition salts of amino groups of the IL-18BP molecule or analogs thereof. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids, such as, for example, hydrochloric acid or sulfuric acid, and salts with organic acids, such as, for example, acetic acid or oxalic acid. Of course, any such salts must retain the biological activity of IL-18BP, e. g. the ability to bind free IL-18.

"Isoforms" of IL-18BP are proteins capable of binding IL-18 or fragment thereof, which may be produced by alternative splicing.

The terms "inhibit", "neutralize" or "block" as used herein, have to be understood as synonyms which mean reducing a molecule, a reaction, an interaction, a gene expression, an mRNA, and/or a protein's expression, stability, function or activity by a measurable amount or to prevent entirely. Inhibitors are compounds that, e.g., bind to, partially or totally block stimulation, decrease, prevent, delay activation, inactivate, desensitize, or down regulate a protein, a gene, and an mRNA stability, expression, function and activity, e.g., antagonists.

The term "Chimeric Antigen Receptor" or alternatively a "CAR" refers to a set of polypeptides, typically two in the simplest embodiments, which when in an immune effector cell, provides the cell with specificity for a target cell, typically a cancer cell, and with intracellular signal generation. In some embodiments, a CAR comprises at least an extracellular antigen binding domain, a transmembrane domain and a cytoplasmic signaling domain (also referred to herein as "an intracellular signaling domain") comprising a functional signaling domain derived from a stimulatory molecule and/or costimulatory molecule as defined below. In some embodiments, the set of polypeptides are contiguous with eachother. In some embodiments, the set of polypeptides include a dimerization switch that, upon the presence of a dimerization molecule, can couple the polypeptides to one another, e.g., can couple an antigen binding domain to an intracellular signaling domain. In one aspect, the stimulatory molecule is the zeta chain associated with the T cell receptor complex. In one embodiment, the cytoplasmic signaling domain further comprises one or more functional signaling domains derived from at least one costimulatory molecule as defined below. In one aspect, the costimulatory molecule is chosen from the costimulatory molecules described herein, e.g., 4-1BB (i.e., CD137), CD27 and/or CD28. In one embodiment, the CAR comprises a chimeric fusion protein comprising an extracellular antigen binding domain, a transmembrane domain and an intracellular signaling domain comprising a functional signaling domain derived from a stimulatory molecule. In one embodiment, the CAR comprises a chimeric fusion protein comprising an extracellular antigen binding domain, a transmembrane domain and an intracellular signaling domain comprising a functional signaling domain derived from a costimulatory molecule and a functional signaling domain derived from a stimulatory molecule. In one embodiment, the CAR comprises a chimeric fusion protein comprising an extracellular antigen binding domain, a transmembrane domain and an intracellular signaling domain comprising two functional signaling domains derived from one or more costimulatory molecule(s) and a functional signaling domain derived from a stimulatory molecule. In one embodiment, the CAR comprises a chimeric fusion protein comprising an extracellular antigen binding domain, a transmembrane domain and an intracellular signaling domain comprising at least two functional signaling domains derived from one or more costimulatory molecule(s) and a functional signaling domain derived from a stimulatory molecule. In one embodiment, the CAR comprises an optional leader sequence at the amino-terminus (N-ter) of the CAR fusion protein. In one embodiment, the CAR further comprises a leader sequence at the N-terminus of the extracellular antigen binding domain, wherein the leader sequence is optionally cleaved from the antigen binding domain (e.g., a scFv) during cellular processing and localization of the CAR to the cellular membrane.

A CAR that comprises an antigen binding domain (e.g., a scFv, or TCR) that targets a specific tumor maker X, such as those described herein, is also referred to as XCAR. For example, a CAR that comprises an antigen binding domain that targets CD 19 is referred to as CD19CAR.

The term "signaling domain" refers to the functional portion of a protein which acts by transmitting information within the cell to regulate cellular activity via defined signaling pathways by generating second messengers or functioning as effectors by responding to such messengers.

The portion of the CAR comprising an antibody or antibody fragment thereof may exist in a variety of forms where the antigen binding domain is expressed as part of a contiguous polypeptide chain including, for example, a single domain antibody fragment (sdAb), a single chain antibody (scFv), a humanized antibody or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition of the invention comprises an antibody fragment. In a further aspect, the CAR comprises an antibody fragment that comprises a scFv. The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof.

The term "antibody fragment" refers to at least one portion of an antibody, that retains the ability to specifically interact with (e.g., by binding, steric hinderance, stabilizing/destabilizing, spatial distribution) an epitope of an antigen. Examples of antibody fragments include, but are not limited to, Fab, Fab', F(ab')2, Fv fragments, scFv antibody fragments, disulfide-linked Fvs (sdFv), a Fd fragment consisting of the VH and CHI domains, linear antibodies, single domain antibodies such as sdAb (either VL or VH), camelid VHH domains, multi- specific antibodies formed from antibody fragments such as a bivalent fragment comprising two Fab fragments linked by a disulfide brudge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23: 1126-1136, 2005). Antigen binding fragments can also be grafted into scaffolds based on polypeptides such as a fibronectin type III (Fn3)(see U.S. Patent No.: 6,703,199, which describes fibronectin polypeptide minibodies).

The term "scFv" refers to a fusion protein comprising at least one antibody fragment comprising a variable region of a light chain and at least one antibody fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

The portion of the CAR comprising an antibody or antibody fragment thereof may exist in a variety of forms where the antigen binding domain is expressed as part of a contiguous polypeptide chain including, for example, a single domain antibody fragment
(sdAb), a single chain antibody (scFv), a humanized antibody, or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition of the invention comprises an antibody fragment. In a further aspect, the CAR comprises an antibody fragment that comprises a scFv.

As used herein, the term "binding domain" or "antibody molecule" refers to a protein, e.g., an immunoglobulin chain or fragment thereof, comprising at least one immunoglobulin variable domain sequence. The term "binding domain" or "antibody molecule" encompasses antibodies and antibody fragments. In an embodiment, an antibody molecule is a multispecific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

The term "antibody heavy chain," refers to the larger of the two types of polypeptide chains present in antibody molecules in their naturally occurring conformations, and which normally determines the class to which the antibody belongs.

The term "antibody light chain," refers to the smaller of the two types of polypeptide chains present in antibody molecules in their naturally occurring conformations. Kappa (κ) and lambda (λ) light chains refer to the two major antibody light chain isotypes.

The term "recombinant antibody" refers to an antibody which is generated using recombinant DNA technology, such as, for example, an antibody expressed by a bacteriophage or yeast expression system. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using recombinant DNA or amino acid sequence technology which is available and well known in the art.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be generated synthesized or can be derived from a biological sample, or might be macromolecule besides a polypeptide. Such a biological sample can include, but is not limited to a tissue sample, a tumor sample, a cell or a fluid with other biological components.

The term "anti-cancer effect" refers to a biological effect which can be manifested by various means, including but not limited to, e.g., a decrease in tumor volume, a decrease in the number of cancer cells, a decrease in the number of metastases, an increase in life expectancy, decrease in cancer cell proliferation, decrease in cancer cell survival, or amelioration of various physiological symptoms associated with the cancerous condition. An "anti-cancer effect" can also be manifested by the ability of the peptides, polynucleotides, cells and antibodies in prevention of the occurrence of cancer in the first place. The term "anti-tumor effect" refers to a biological effect which can be manifested by various means, including but not limited to, e.g., a decrease in tumor volume, a decrease in the number of tumor cells, a decrease in tumor cell proliferation, or a decrease in tumor cell survival.

The phrase "disease associated with expression of a tumor antigen as described herein" includes, but is not limited to, a disease associated with expression of a tumor antigen as described herein or condition associated with cells which express a tumor antigen as described herein including, e.g., proliferative diseases such as a cancer or malignancy or a precancerous condition such as a myelodysplasia, a myelodysplastic syndrome or a preleukemia; or a noncancer related indication associated with cells which express a tumor antigen as described herein. In one aspect, a cancer associated with expression of a tumor antigen as described herein is a hematological cancer. In one aspect, a cancer associated with expression of a tumor antigen as described herein is a solid cancer. Further diseases associated with expression of a tumor antigen described herein include, but not limited to, e.g., atypical and/or non-classical cancers, malignancies, precancerous conditions or proliferative diseases associated with expression of a tumor antigen as described herein. Non-cancer related indications associated with expression of a tumor antigen as described herein include, but are not limited to, e.g., autoimmune disease, (e.g., lupus), inflammatory disorders (allergy and asthma) and transplantation. In some embodiments, the tumor antigen-expressing cells express, or at any time expressed, mRNA encoding the tumor antigen. In an embodiment, the tumor antigen -expressing cells produce the tumor antigen protein (e.g., wild-type or mutant), and the tumor antigen protein may be present at normal levels or reduced levels. In an embodiment, the tumor antigen -expressing cells produced detectable levels of a tumor antigen protein at one point, and subsequently produced substantially no detectable tumor antigen protein.

The terms "cancer associated antigen" or "tumor antigen" interchangeably refer to a molecule (typically a protein, carbohydrate or lipid) that is expressed on the surface of a cancer cell, either entirely or as a fragment (e.g., MHC/peptide), and which is useful for the preferential targeting of a pharmacological agent to the cancer cell. In some embodiments, a tumor antigen is a marker expressed by both normal cells and cancer cells, e.g., a lineage marker, e.g., CD19 on B cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some enbodiments, a tumor antigen is a cell surface molecule that is inappropriately synthesized in the cancer cell, for instance, a molecule that contains deletions, additions or mutations in comparison to the molecule expressed on a normal cell. In some embodiments, a tumor antigen will be expressed exclusively on the cell surface of a cancer cell, entirely or as a fragment (e.g., MHC/peptide), and not synthesized or expressed on the surface of a normal cell. In some embodiments, the CARs of the present invention includes CARs comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus -specific and/or tumor-specific peptide/MHC complexes represent a unique class of cell surface targets for immunotherapy. TCR-like antibodies targeting peptides derived from viral or tumor antigens in the context of human leukocyte antigen (HLA)-AI or HLA-A2 have been described (see, e.g., Sastry et al., J Virol. 2011 85(5): 1935-1942; Sergeeva et al., Blood, 2011 117(16):4262-4272; Verma et al., J Immunol 2010 184(4):2156-2165; Willemsen et al., Gene Ther 2001 8(21): 1601-1608 ; Dao et al., Sci Transl Med 2013 5(176) : 176ra33 ; Tassev et al., Cancer Gene Ther 2012 19(2):84-100). For example, TCR-like antibody can be identified from screening a library, such as a human scFv phage displayed library.

In the context of the present invention, "tumor antigen" or "hyperproliferative disorder antigen" or "antigen associated with a hyperproliferative disorder" refers to antigens that are common to specific hyperproliferative disorders, such as cancer.

### BRIEF DESCRIPTION OF THE SEQUENCES

SEQ ID NO 1: 13-amino acid Linker Sequence of hIL-18BP:
   Glu-Phe-Gly-Ala-Gly-Leu-Val-Leu-Gly-Gly-Gln-Phe-Met

SEQ ID NO 2: Amino Acid Sequence of IL-18 Binding Protein (hIL-18BP), isoform a

TPVSQTTTAA TASVRSTKDP CPSQPPVFPA AKQCPALEVT WPEVEVPLNG
TLSLSCVACS RFPNFSILYW LGNGSFIEHL PGRLWEGSTS RERGSTGTQL CKALVLEQLT
PALHSTNFSC VLVDPEQVVQ RHVVLAQLWA GLRATLPPTQ EALPSSHSSP QQQG

SEQ ID NO 3: Amino Acid Sequence of IL-18 Binding Protein (hIL-18BP), isoform b

TPVSQTTTAA TASVRSTKDP CPSQPPVFPA AKQCPALEVT WPEVEVPL SWAEGNLAPH
PRSPALQPQQ STAAGLRLST GPAAAQP

SEQ ID NO: 4: Amino Acid Sequence of IL-18 Binding Protein (hIL-18BP), isoform c

TPVSQTTTAA TASVRSTKDP CPSQPPVFPA AKQCPALEVT WPEVEVPLNG
TLSLSCVACS RFPNFSILYW LGNGSFIEHL PGRLWEGSTS RERGSTGTQL CKALVLEQLT
PALHSTNFSC VLVDPEQVVQ RHVVLAQLWV RSPRRGLQEQ EELCFHMWGK
GGLCQSSL

SEQ ID NO: 5: Amino Acid Sequence of IL-18 Binding Protein (hIL-18BP), isoform d

TPVSQTTTAA TASVRSTKDP CPSQPPVFPA AKQCPALEVT WPEVEVPLNG
TLSLSCVACS RFPNFSILYW LGNGSFIEHL PGRLWEGSTS RERGSTGWAE
GNLAPHPRSP ALQPQQSTAA GRLSTGPAAA QP

### Examples

Generation of transgenically encoded mouse Il-18 in CAR T cells in the treatment of murine B16F10 melanoma. Efficacy and IL-18 driven cytokine release syndrome.

IL-18 signaling enhances TCR signaling and therefore it was concluded that it could also potentiate the CAR T activation/proliferation since the CAR T contains the TCR signaling domain for T cell activation and proliferation. A note of caution thogh is that the increased CAR T activation may lead to exaggerated antigen-specific T cell activation and also to Ag-nonspecific activation mimicking a hyeprinflammatoy status similar to cytokine releases syndrome.

### Methods

Murine T cells are engineered to express murine IL-18-expressing and anti-mouse CD19 (murine CD19-IL-18). Murine CD19-IL-18 CAR T cells are adoptively transferred to C57BL/6 mice beating B16F10 melanoma tumors expressing CD19. Co-expression of IL-18 in CAR T cells results in the development of a T-bet^{high} phenotype and the stimulation of a systemic acute inflammatory response.

It has been previously observed that one week after cell transfer the mice show very high levels of IL-18 in blood coincident with the CAR T cell expansion, and that decreases afterwards. Coincident with the high levels of IL-18 the mouse body weight decreased as the main manifestation of cytokine release syndrome together with mild manifestations of systemic inflammation.

Strategies to minimize detrimental side effects represent an appealing approach to use this therapy in solid tumors without harmful complications in humans.

The administration of rIL-18BP in the days following the CAR T cell transfer surprisingly represents a safe way to avoid detrimental toxic effects. In this regard, IL-18BP may be administered at a dose of 1mg/kg to 3 mg/kg i.v. in a saline solution upon development of side effects or, e.g., upon determining elevated IL-18 levels in the patient.

## Claims

1. An IL-18 binding molecule for use in a Chimeric Antigen Receptor T-cell (CAR-T) therapy of a patient diagnosed with a disease associated with expression of a tumor antigen to avoid or minimize side effects related to the CAR-T therapy, in particular as a modulator of IL-18 levels in the patient to avoid or minimize side effects related to the CAR-T therapy.

2. The IL-18 binding molecule for use of claim 1, wherein the disease associated with expression of a tumor antigen is a proliferative disease, a precancerous condition, a cancer, and/or a non-cancer related indication associated with expression of the tumor antigen, in particular wherein the patient has cancer, particularly a hematological cancer or a solid tumor, particularly a tumor expressing tumor-associated antigens.

3. The IL-18 binding molecule for use of claim 2, wherein the cancer is a hematologic cancer selected from one or more of chronic lymphocytic leukemia (CLL), acute leukemia, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative condition, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, and Waldenstrom macroglobulinemia.

4. The IL-18 binding molecule for use of any one of claims 1 to 3, wherein a population of cells is used in the CAR-T therapy, which are engineered to express a chimeric antigen receptor (CAR), wherein the CAR comprises an antigen-binding domain, a transmembrane domain, and an intracellular signaling domain, preferably wherein the cells are further engineered to express IL-18.

5. The IL-18 binding molecule for use of any one of claims 1 to 4, wherein the side effects are due to an overshooting of the immune system and/or a cytokine release syndrome, particularly wherein the side effects are selected from the group consisting of unremitting fever, hypotension, hypoxia (lung involvement), other end-organ damage and dysfunction including but not limited to liver and kidney, cytopenias, coagulopathy, hemophagocytosis and neurologic toxicities.

6. The IL-18 binding molecule for use of any one of claims 1 to 5, which is
(a) an IL-18 binding protein (IL-18BP), including any functional equivalent or functional part thereof which retains the modulator activity, in particular a human IL-18BP (hIL-18 BP), preferably a recombinant human IL-18BP (rhlL-18 BP), including any functional equivalent or functional part thereof, which retains the modulator activity; or
(b) an IL-18 binding antibody that specifically binds to free IL-18, but not to IL-18 bound in a complex, including any functional equivalent or functional part thereof which retains the modulator activity.

7. The IL-18 binding molecule for use of claim 6(a), wherein said human IL-18BP is selected from isoform a, b, c and d of human IL-18BP, particularly isoform a as in SEQ ID NO: 2, isoform b as in SEQ ID NO: 3, isoform c as in SEQ ID NO: 4 or isoform d as in SEQ ID NO: 5, including any functional equivalent or functional part of isoforms a, b, c and/or d, which retains the modulator activity, in particular an IL-18BP as shown in SEQ ID NO: 2, including any functional equivalent or functional part thereof, which retains the modulator activity, preferably wherein the functional equivalent has a sequence identity of 80%, 85%, 90%, 91%, 92%, 93%, 94%, 95%, 96%, 97%, 98%, 99% to the sequence depicted in SEQ ID NO: 2 and retains the modulator activity, in particular wherein the functional equivalent or functional part thereof includes a mutein of IL-18BP, a fragment, a peptide, a functional derivative, a functional fragment, a fraction, a circularly permuted derivative, a fused protein comprising IL-18BP, an isoform or a salt thereof which retains the modulator activity.

8. A composition comprising the IL-18 binding molecule of claims 6 or 7 for use of any one of claims 1 to 5.

9. The IL-18 binding molecule for use of claim 2 or the composition for use of claim 8, wherein the cancer is a solid tumor, particularly selected from the group consisting of colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancer, combinations of said cancers, and metastatic lesions of said cancers.

10. The IL-18 binding molecule for use of any one of claims 1 to 7 or 9, or the composition for use of any one of claims 8 or 9, wherein in a body fluid of the patient an abnormal level of free IL-18 has been determined, in particular wherein the level of free IL-18 exceeds the level of free IL-18 in body fluids of a healthy control subject, particularly by 5%, 10%, 20%, 30%, 40%, 50%, 60%, 70%, 80%, 90%, 100%, or more than 100%, using an assay capable of detecting free IL-18 in body fluids, said assay comprising the IL-18 binding molecule as defined in any one of claims 6 or 7.

11. The IL-18 binding molecule for use, the composition for use of claim 10, wherein the assay for quantifying the level of free IL-18 in the body fluid(s) comprises the following steps:
a) bringing a sample of body fluid suspected to contain free IL-18 into contact with the IL-18 binding molecule as defined in any one of claims 6 or 7 as the capturing molecule for free IL-18;
b) allowing the IL-18 binding molecule to bind to free IL-18; and
c) detecting the binding of the IL-18 binding molecule and determining the amount of free IL-18 in the sample.

12. The IL-18 binding molecule for use, the composition for use of any one of claims 10 or 11, wherein the body fluid is selected from the group consisting of broncho-alveolar lavage fluid (BALF) circulation fluids, secretion fluids, biopsy, and homogenized tissue, particularly serum, urine, tear, saliva, bile, sweat, exhalation or expiration, sputum, bronchoalveolar fluid, sebum, cellular, gland, mucosa and tissue secretion.

13. The IL-18 binding molecule for use of any one of claims 1 to 7 or 9 to 12, or the composition for use of any one of claims 8 to 12, wherein the therapy of the patient further comprises the use of one or more check-point inhibitor(s).

14. The IL-18 binding molecule for use of claim 13 or the composition for use of claim 13, wherein the check-point inhibitor is a CTLA-4 inhibitor, a PD-1 inhibitor or a PD-L1 inhibitor.

15. The IL-18 binding molecule for use of any one of claims 1 to 7 or 9 to 14, or the composition for use of any one of claims 8 to 14, wherein the CAR-T and the IL-18 binding molecule or the composition, respectively, are administered to the patient concomitantly or sequentially, or wherein the IL-18 binding molecule or the composition, respectively, is administered to the patient as an acute intervention during the CAR-T therapy.


SEQUENCE LISTING

<110> AB2 BIO SA

<120> CAR-T CELL THERAPY

<130> AD1615 EP BS

<160> 5

<170> BiSSAP 1.3.6

<210> 1
<211> 13
<212> PRT
<213> Artificial Sequence

<220>
<223> 13-amino acid Linker Sequence of hIL-18BP

<400> 1
Glu Phe Gly Ala Gly Leu Val Leu Gly Gly Gln Phe Met
1               5                   10

<210> 2
<211> 164
<212> PRT
<213> Mammalia

<220>
<223> Amino Acid Sequence of IL-18 Binding Protein (hIL-18BP)

<400> 2
Thr Pro Val Ser Gln Thr Thr Thr Ala Ala Thr Ala Ser Val Arg Ser
1               5                   10                  15
Thr Lys Asp Pro Cys Pro Ser Gln Pro Pro Val Phe Pro Ala Ala Lys
            20                  25                  30
Gln Cys Pro Ala Leu Glu Val Thr Trp Pro Glu Val Glu Val Pro Leu
        35                  40                  45
Asn Gly Thr Leu Ser Leu Ser Cys Val Ala Cys Ser Arg Phe Pro Asn
    50                  55                  60
Phe Ser Ile Leu Tyr Trp Leu Gly Asn Gly Ser Phe Ile Glu His Leu
65                  70                  75                  80
Pro Gly Arg Leu Trp Glu Gly Ser Thr Ser Arg Glu Arg Gly Ser Thr
                85                  90                  95
Gly Thr Gln Leu Cys Lys Ala Leu Val Leu Glu Gln Leu Thr Pro Ala
            100                 105                 110
Leu His Ser Thr Asn Phe Ser Cys Val Leu Val Asp Pro Glu Gln Val
        115                 120                 125
Val Gln Arg His Val Val Leu Ala Gln Leu Trp Ala Gly Leu Arg Ala
    130                 135                 140
Thr Leu Pro Pro Thr Gln Glu Ala Leu Pro Ser Ser His Ser Ser Pro
145                 150                 155                 160
Gln Gln Gln Gly

<210> 3
<211> 85
<212> PRT
<213> Mammalia

<220>
<223> Amino Acid Sequence of IL-18 Binding Protein (hIL-18BP)

<400> 3
Thr Pro Val Ser Gln Thr Thr Thr Ala Ala Thr Ala Ser Val Arg Ser
1               5                   10                  15
Thr Lys Asp Pro Cys Pro Ser Gln Pro Pro Val Phe Pro Ala Ala Lys
            20                  25                  30
Gln Cys Pro Ala Leu Glu Val Thr Trp Pro Glu Val Glu Val Pro Leu
        35                  40                  45
Ser Trp Ala Glu Gly Asn Leu Ala Pro His Pro Arg Ser Pro Ala Leu
    50                  55                  60
Gln Pro Gln Gln Ser Thr Ala Ala Gly Leu Arg Leu Ser Thr Gly Pro
65                  70                  75                  80
Ala Ala Ala Gln Pro
                85

<210> 4
<211> 168
<212> PRT
<213> Mammalia


<220>
<223> Amino Acid Sequence of IL-18 Binding Protein (hIL-18BP)

<400> 4
Thr Pro Val Ser Gln Thr Thr Thr Ala Ala Thr Ala Ser Val Arg Ser
1               5                   10                  15
Thr Lys Asp Pro Cys Pro Ser Gln Pro Pro Val Phe Pro Ala Ala Lys
            20                  25                  30
Gln Cys Pro Ala Leu Glu Val Thr Trp Pro Glu Val Glu Val Pro Leu
        35                  40                  45
Asn Gly Thr Leu Ser Leu Ser Cys Val Ala Cys Ser Arg Phe Pro Asn
    50                  55                  60
Phe Ser Ile Leu Tyr Trp Leu Gly Asn Gly Ser Phe Ile Glu His Leu
65                  70                  75                  80
Pro Gly Arg Leu Trp Glu Gly Ser Thr Ser Arg Glu Arg Gly Ser Thr
                85                  90                  95
Gly Thr Gln Leu Cys Lys Ala Leu Val Leu Glu Gln Leu Thr Pro Ala
            100                 105                 110
Leu His Ser Thr Asn Phe Ser Cys Val Leu Val Asp Pro Glu Gln Val
        115                 120                 125
Val Gln Arg His Val Val Leu Ala Gln Leu Trp Val Arg Ser Pro Arg
    130                 135                 140
Arg Gly Leu Gln Glu Gln Glu Glu Leu Cys Phe His Met Trp Gly Lys
145                 150                 155                 160
Gly Gly Leu Cys Gln Ser Ser Leu
                165

<210> 5
<211> 132
<212> PRT
<213> Mammalia


<220>
<223> Amino Acid Sequence of IL-18 Binding Protein (hIL-18BP)

<400> 5
Thr Pro Val Ser Gln Thr Thr Thr Ala Ala Thr Ala Ser Val Arg Ser
1               5                   10                  15

Thr Lys Asp Pro Cys Pro Ser Gln Pro Pro Val Phe Pro Ala Ala Lys
            20                  25                  30
Gln Cys Pro Ala Leu Glu Val Thr Trp Pro Glu Val Glu Val Pro Leu
        35                  40                  45
Asn Gly Thr Leu Ser Leu Ser Cys Val Ala Cys Ser Arg Phe Pro Asn
    50                  55                  60
Phe Ser Ile Leu Tyr Trp Leu Gly Asn Gly Ser Phe Ile Glu His Leu
65                  70                  75                  80
Pro Gly Arg Leu Trp Glu Gly Ser Thr Ser Arg Glu Arg Gly Ser Thr
                85                  90                  95
Gly Trp Ala Glu Gly Asn Leu Ala Pro His Pro Arg Ser Pro Ala Leu
            100                 105                 110
Gln Pro Gln Gln Ser Thr Ala Ala Gly Arg Leu Ser Thr Gly Pro Ala
        115                 120                 125
Ala Ala Gln Pro
    130